# FASCICULE DE BREVET EUROPEEN

(11) **EP 0 793 639 B1**
(45) Date de publication et mention de la délivrance du brevet: **30.12.1998**
(21) Numéro de dépôt: 95932796.6
(22) Date de dépôt: 27.09.1995
(51) Int. Cl.: C07C 205/56, C07C 205/55, C07C 201/12

(54) **PROCEDE DE PREPARATION DE DERIVES DU NITROBENZENE**
VERFAHREN ZUR HERSTELLUNG VON NITROBENZOLDERIVATEN
METHOD FOR PREPARING NITROBENZENE DERIVATIVES

(30) Priorité: 29.09.1994 FR 9411657
(43) Date de publication de la demande: 10.09.1997
(73) Titulaire: RHONE-POULENC RORER S.A., 92160 Antony (FR)
(72) Inventeur: MANFRE, Franco, F-94460 Limeil-Brevannes (FR)
(74) Mandataire: Savina, Jacques
(86) Numéro de dépôt international: FR9501241
(87) Numéro de publication internationale: WO9610010

(56) Documents cités:
- CH-A- 480 072
- US-A- 4 659 862
- US-A- 4 859 232
- JOURNAL OF ORGANIC CHEMISTRY, vol. 42, no. 22, 1977 EASTON US, pages 3491-3494, N.A. CORTESE ET AL. 'Palladium-Catalyzed Reductions of Halo- and Nitroaromatic Compounds with Triethylammonium Formate' cité dans la demande

## Description

La présente invention concerne un procédé de préparation de dérivés du nitrobenzène de formule : 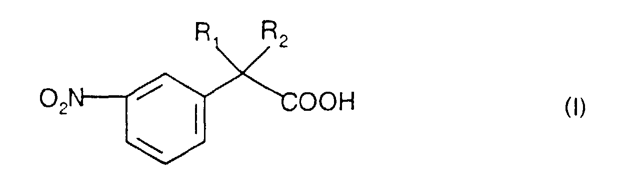 dans laquelle R₁ et R₂, identiques ou différents, représentent un atome d'hydrogène ou un radical alkyle contenant 1 à 6 atomes de carbone en chaîne droite ou ramifiée ou alcoxy contenant 1 à 6 atomes de carbone en chaîne droite ou ramifiée ou bien R₁ et R₂ forment avec l'atome de carbone auquel ils sont rattachés un radical cycloalkyle contenant 3 à 6 atomes de carbone qui sont notamment utiles comme intermédiaires pour la préparation de produits pharmaceutiques (brevet WO 91/12264) ou comme antidotes pour certains insecticides (brevet US 4859232).

Il est connu de préparer ces composés par hydrolyse acide des nitriles correspondants eux-mêmes obtenus à partir des dérivés halogénés correspondants et d'un cyanure de métal alcalin (E. FELDER, J. Med. Chem., 13, 559 (1970) et brevet US 4859232). L'utilisation de cyanure de métal alcalin peut poser des problèmes de pollution au niveau industriel.

Par ailleurs, il est connu de déhalogéner des halogénonitrobenzènes au moyen de formiate de triéthylammonium, en présence de palladium sur charbon, en milieu clos, sans solvant et à une température de 100°C (N.A. CORTESE et coll., J. Org. Chem., 42, 22, 3491-3494 (1977). Du fait d'opérer en milieu clos et à 100°C, ce procédé est difficilement industrialisable. De plus, appliqué à la préparation des composés de formule (I) ce procédé conduit à un mélange de 3 produits (produit de formule (I) attendu, produit de départ et produit déhalogéné mais dont le nitro est réduit en amino) qui sont difficiles à séparer et le rendement en produit attendu est faible.

Il a maintenant été trouvé un procédé industriel non polluant permettant d'obtenir les composés de formule (I) purs et avec de bons rendements.

Ce procédé consiste à déhalogéner un dérivé de formule : 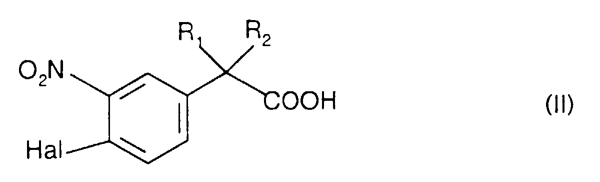 dans laquelle R₁ et R₂ ont les mêmes significations que dans la formule (I) et Hal représente un atome de chlore ou de brome, sous pression atmosphérique, au moyen de 1 à 2 mole de formiate de triéthylammonium, en présence de 0,002 à 0,1 mole de palladium sur charbon pour une mole de composé de formule (Il), au sein de l'acétonitrile ou du tétrahydrofuranne, à une température comprise entre 50°C et la température d'ébullition du milieu réactionnel.

Cette réaction s'effectue de préférence sous atmosphère inerte (azote par exemple).

Il est particulièrement avantageux d'utiliser 1,5 mole de formiate de triéthylammonium pour 1 mole de dérivé de formule (Il).

Le formiate de triéthylammonium peut être formé in situ à partir de 2 équivalents molaires de triéthylamine et de 1,5 équivalent molaire d'acide formique.

De préférence, on utilise du palladium sur charbon à 5% et, en particulier, 0,01 mole de palladium sur charbon à 5% par mole de composé (Il).

La quantité préférée d'acétonitrile ou de tétrahydrofuranne est comprise entre 5 à 15 équivalents massiques par rapport au composé de formule (Il) et, en particulier 10 équivalents.

La température réactionnelle est de préférence voisine de 80°C.

Les dérivés de formule (Il) peuvent être obtenus par application ou adaptation du procédé décrit dans le brevet DE 22100418 ou par nitration des dérivés halogénés correspondants comme décrit dans les exemples.

Les exemples suivants illustrent l'invention.

### Exemple 1

Dans un ballon, sous atmosphère inerte, contenant 67,7 g d'acide (chloro-4 nitro-3 phényl)-2 propionique-(RS), 87 ml de triéthylamine et 6,1 g de palladium sur charbon à 5% dans 680 ml d'acétonitrile, on ajoute en 5 minutes 16,5 ml d'acide formique. Le milieu réactionnel est chauffé au reflux pendant 18 heures puis après retour à une température voisine de 20°C filtré sur célite. Le filtrat est évaporé à sec sous pression réduite et repris dans 500 ml d'eau et 400 ml d'acétate d'éthyle. La phase organique est décantée et la phase aqueuse extraite par 2 fois 200 ml d'acétate d'éthyle. Les phases organiques réunies sont séchées sur sulfate de magnésium et concentrées à sec sous pression réduite à 40°C. On obtient ainsi 53 g d'acide (nitro-3 phényl)-2 propionique sous forme d'une huile brune [spectre IR (CH₂Cl₂, cm⁻¹) ^{γ}OH acide: 3250-2450; ^{γ}CH Aliph.: 2985-2940; ^{γ}C=O:1715 ;^{γa} NO2 ; 1535; ^{γS} NO2 ; 13551; ^{γ} Ph m subst.: 690; Spectre RMN (DMSO-d6, 200MHz): 1,45 (d, J = 7 Hz, 3H); 3,9 (q, J = 7 Hz, 1H); 7,6 (t, J = 7 Hz, 1H); 7,8 (d, J = 7 Hz, 1H); 8,1 (d, J = 7 Hz, 1H); 8,15 (s, 1H); 12,5 (large s, OH acide)].

L'acide (chloro-4 nitro-3 phényl)-2 propionique peut être préparé selon le brevet DE 2210418.

### Exemple 2

On opère d'une manière analogue à celle décrite à l'exemple 1 mais à partir de 6,3 g d'acide (chloro-4 nitro-3 phényl)-2 méthyl-2 propionique, de 7,62 ml de triéthylamine, de 0,6 g de palladium sur charbon à 5% et de 1,46 ml d'acide formique dans 100 ml d'acétonitrile. On obtient ainsi 5,0 g d'acide (nitro-3 phényl)-2 méthyl-2 propionique sous forme d'une huile orange [Spectre RMN (DMSO-d6, 200MHz): 1,57 (s, 6H); 7,65 (t, J = 7 Hz, 1H); 7,9 (d, J = 7 Hz, 1H); 8,13 (d, J = 7 Hz, 1H); 8,15 (s, 1H); 12,5 (large s, OH acide)].

L'acide (chloro-4 nitro-3 phényl)-2 méthyl-2 propionique peut être préparé de la manière suivante : à une suspension de 5,35 g d'acide (chloro-4 phényl)-2 méthyl-2 propionique dans 50 ml d'acide sulfurique concentré, refroidie à une température voisine de 5°C, on ajoute goutte à goutte une solution contenant 2,65 g de nitrate de potassium dans 10 ml d'acide sulfurique concentré en maintenant le milieu réactionnel à cette même température. L'agitation est poursuivie pendant une heure puis le mélange réactionnel est transvasé, sous agitation, sur 250 g de glace pilée. Le solide blanc obtenu est séparé par filtration, lavé par 2 fois 100 ml d'eau et séché à l'air. On obtient ainsi 6,4g d'acide (chloro-4 nitro-3 phényl)-2 méthyl-2 propionique fondant à 157°C.

### Exemple comparatif selon le procédé N.A. CORTESE.

Un ballon, maintenu sous atmosphère inerte, contenant 1 g d'acide (chloro-4 nitro-3 phényl)-2 propionique-(RS), 0,9 ml de triéthylamine, 90 mg de palladium sur charbon à 5% et 0,2 ml d'acide formique est chauffé pendant 4 heures sous agitation à une température voisine de 100°C. Après retour à une température voisine de 20°C, le mélange réactionnel épais est repris dans 20 ml de CH₂Cl₂ puis filtré sur célite. Le filtrat est lavé successivement par 10 ml d'une solution aqueuse N d'acide chlorhydrique, 10 ml d'eau, séché sur sulfate de magnésium et concentré à sec sous pression réduite à 40°C. On obtient ainsi 0,97g d'une huile marron mélange d'acide (chloro-4 nitro-3 phényl)-2 propionique-(RS), d'acide (nitro-3 phényl)-2 propionique-(RS) et d'acide (amino-3 phényl)-2 propionique-(RS) dans les rapports molaires respectifs de 25, 70 et 3 (exprimés en pourcentage).

## Revendications

1. Procédé de préparation de dérivés du nitrobenzène de formule : 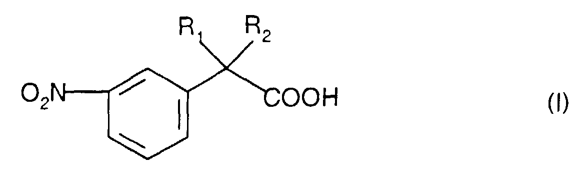 dans laquelle R₁ et R₂, identiques ou différents, représentent un atome d'hydrogène ou un radical alkyle contenant 1 à 6 atomes de carbone en chaîne droite ou ramifiée ou alcoxy contenant 1 à 6 atomes de carbone en chaîne droite ou ramifiée ou bien R₁ et R₂ forment avec l'atome de carbone auquel ils sont rattachés un radical cycloalkyle contenant 3 à 6 atomes de carbone caractérisé en ce que l'on déhalogène un dérivé de formule : 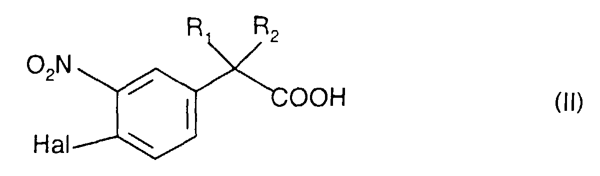 dans laquelle R₁ et R₂ ont les mêmes significations que dans la formule (I) et Hal représente un atome de chlore ou de brome, sous pression atmosphérique, au moyen de 1 à 2 mole de formiate de triéthylammonium, en présence de 0,002 à 0,1 mole de palladium sur charbon pour une mole de composé de formule (II), au sein de l'acétonitrile ou du tétrahydrofuranne, à une température comprise entre 50°C et la température d'ébullition du milieu réactionnel.

2. Procédé selon la revendication 1 caractérisé en ce que l'on opère sous atmosphère inerte.

3. Procédé selon l'une des revendications 1 ou 2 caractérisé en ce que l'on utilise 1,5 mole de formiate de triéthylammonium pour 1 mole de dérivé de formule (Il).

4. Procédé selon l'une des revendication 1 à 3 caractérisé en ce que le formiate de triéthylammonium est formé in situ.

5. Procédé selon l'une des revendications 1 à 4 caractérisé en ce que le formiate d'ammonium est préparé à partir de 2 équivalents molaires de triéthylamine et de 1,5 équivalent molaire d'acide formique.

6. Procédé selon l'une des revendications 1 à 5 caractérisé en ce que l'on utilise du palladium sur charbon à 5%.

7. Procédé selon l'une des revendications 1 à 6 caractérisé en ce que l'on utilise 0,01 mole de palladium sur charbon à 5% par mole de composé (Il).

8. Procédé selon l'une des revendications 1 à 7 caractérisé en ce que l'on utilise entre 5 et 15 équivalents massiques d'acétonitrile ou de tétrahydrofuranne par rapport au composé de formule (II)

9. Procédé selon la revendication 8 caractérisé en ce que l'on utilise 10 équivalents d'acétonitrile ou de tétrahydrofuranne.

10. Procédé selon l'une des revendications 1 à 9 caractérisé en ce que l'on opère à une température voisine de 80°C.

## Patentansprüche

1. Verfahren zur Herstellung von Nitrobenzolderivaten der Formel 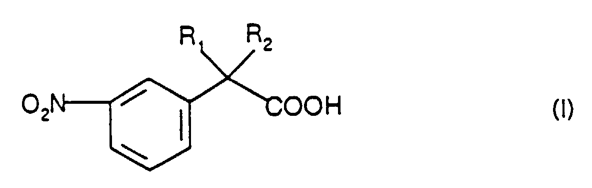 worin R₁ und R₂, gleich oder voneinander verschieden, ein Wasserstoffatom oder einen Alkylrest mit 1 bis 6 Kohlenstoffatomen in gerader oder verzweigter Kette oder Alkoxy mit 1 bis 6 Kohlenstoffatomen in gerader oder verzweigter Kette bedeuten oder aber R₁ und R₂ mit dem Kohlenstoffatom, an das sie gebunden sind, einen Cycloalkylrest mit 3 bis 6 Kohlenstoffatomen bilden,
dadurch gekennzeichnet, daß man ein Derivat der Formel 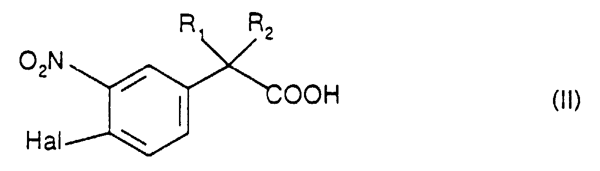 worin R₁ und R₂ die gleichen Bedeutungen wie für Formel (I) angegeben besitzen und Hal ein Chlor- oder Bromatom darstellt, unter Atmosphärendruck mittels 1 bis 2 Mol Triethylammoniumformiat in Anwesenheit von 0,002 bis 0,1 Mol Palladium-auf-Aktivkohle je 1 Mol Verbindung der Formel (II) in Acetonitril oder Tetrahydrofuran bei einer Temperatur zwischen 50°C und der Siedetemperatur des Reaktionsmilieus dehalogeniert.

2. Verfahren gemäß Anspruch 1, dadurch gekennzeichnet, daß man unter inerter Atmosphäre arbeitet.

3. Verfahren gemäß einem der Ansprüche 1 oder 2, dadurch gekennzeichnet, daß man 1,5 Mol Triethylammoniumformiat je 1 Mol Derivat der Formel (II) verwendet.

4. Verfahren gemäß einem der Ansprüche 1 bis 3, dadurch gekennzeichnet, daß das Triethylammoniumformiat in situ gebildet wird.

5. Verfahren gemäß einem der Ansprüche 1 bis 4, dadurch gekennzeichnet, daß das Ammoniumformiat aus 2 Moläquivalenten Triethylamin und 1,5 Moläquivalenten Ameisensäure gebildet wird.

6. Verfahren gemäß einem der Ansprüche 1 bis 5, dadurch gekennzeichnet, daß man Palladium zu 5% auf Aktivkohle verwendet.

7. Verfahren gemäß einem der Ansprüche 1 bis 6, dadurch gekennzeichnet, daß man 0,01 Mol Palladium zu 5% auf Aktivkohle je Mol Verbindung (II) verwendet.

8. Verfahren gemäß einem der Ansprüche 1 bis 7, dadurch gekennzeichnet, daß man zwischen 5 und 15 Volumenäquivalenten Acetonitril oder Tetrahydrofuran in bezug auf die Verbindung der Formel (II) einsetzt.

9. Verfahren gemäß Anspruch 8, dadurch gekennzeichnet, daß man 10 Äquivalente Acetonitril oder Tetrahydrofuran einsetzt.

10. Verfahren gemäß einem der Ansprüche 1 bis 9, dadurch gekennzeichnet, daß man bei einer Temperatur von etwa 80°C arbeitet.

## Claims

1. Process for the preparation of nitrobenzene derivatives of formula: 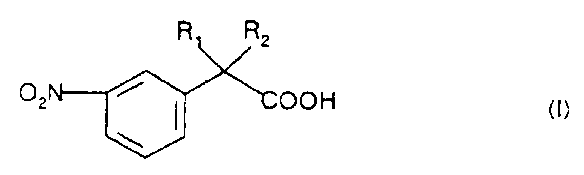 in which R₁ and R₂, which may be identical or different, represent a hydrogen atom or an alkyl radical containing 1 to 6 straight-chain or branched-chain carbon atoms or an alkoxy radical containing 1 to 6 straight-chain or branched-chain carbon atoms, or alternatively R₁ and R₂, together with the carbon atom to which they are attached, form a cycloalkyl radical containing 3 to 6 carbon atoms, characterized in that a derivative of formula: 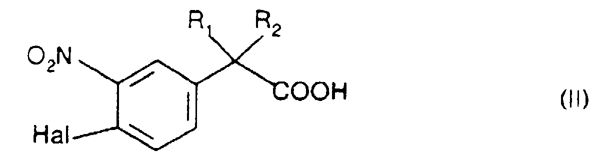 in which R₁ and R₂ have the same meanings as in formula (I) and Hal represents a chlorine or bromine atom, is dehalogenated at atmospheric pressure, using 1 to 2 mol of triethylammonium formate, in the presence of 0.002 to 0.1 mol of palladium-on-charcoal per mole of compound of formula (II), in acetonitrile or tetrahydrofuran, at a temperature between 50°C and the boiling point of the reaction medium.

2. Process according to Claim 1, characterized in that the procedure is performed under inert atmosphere.

3. Process according to either of Claims 1 and 2, characterized in that 1.5 mol of triethylammonium formate are used per 1 mol of derivative of formula (II).

4. Process according to one of Claims 1 to 3, characterized in that the triethylammonium formate is formed in situ.

5. Process according to one of Claims 1 to 4, characterized in that the ammonium formate is prepared from 2 molar equivalents of triethylamine and 1.5 molar equivalents of formic acid.

6. Process according to one of Claims 1 to 5, characterized in that 5% palladium-on-charcoal is used.

7. Process according to one of Claims 1 to 6, characterized in that 0.01 mol of 5% palladium-on-charcoal is used per mole of compound of formula (II).

8. Process according to one of Claims 1 to 7, characterized in that between 5 and 15 weight equivalents of acetonitrile or tetrahydrofuran are used relative to the compound of formula (II).

9. Process according to Claim 8, characterized in that 10 equivalents of acetonitrile or tetrahydrofuran are used.

10. Process according to one of Claims 1 to 9, characterized in that the procedure is performed at a temperature in the region of 80°C.
